# EUROPEAN PATENT APPLICATION

(11) **EP 3 943 945 A1**
(43) Date of publication of application: **26.01.2022**
(21) Application number: 20186936.9
(22) Date of filing: 21.07.2020
(51) Int. Cl.: G01N 33/58, C09B 11/08, C12M 1/00, G01N 15/14

(54) **MULTIPLEXED CELL PHOTOTAGGING**

(71) Applicant: Erasmus University Rotterdam Medical Center, 3015 GE Rotterdam (NL)
(72) Inventor: CHIEN, Miao Ping, 3015 GE Rotterdam (NL)
(74) Representative: V.O.

(57) **Abstract**

A method for distinguishing cells in a sample (S). The sample is provided with photoaffinity tags (T). Each tag (T) comprises a photoreactive moiety (Tb) configured to bind to a nearby cell upon irradiation by photoactivating light (La), and a molecular identification structure (Ti) connected to the photoreactive moiety. A target location (St) of the sample (S) is determined with a target cell (Ct) to be distinguished. The target location (St) is selectively irradiated with the photoactivating light (La) to cause a photoactivated tag (T') to bind with the target cell (Ct) in the target location (St). The target cell (Ct) is distinguished from other cells (C) in the sample (S) by identifying the molecular identification structure (Ti) of the tag (T") bound to the target cell (Ct).

## Description

### TECHNICAL FIELD AND BACKGROUND

The present disclosure relates to methods for distinguishing cells, photoaffinity tags for use in such methods, methods of manufacturing such tags, and systems for implementing the methods and/or using the tags.

As described by Smith and Collins [Future Med Chem. 2015 Feb; 7(2): 159-183] photoaffinity labeling (PAL) is the use of a chemical probe that can covalently bind to its target in response to activation by light. This is made possible by the incorporation of a photoreactive group within an otherwise reversibly binding probe compound. On irradiation with a specific wavelength of light, the photoreactive group forms a reactive intermediate that rapidly reacts with and binds to the nearest molecule, which in the known method is a target protein. The known technique is conventionally used for the study of protein-ligand interactions, where it can identify unknown targets of ligands, assist in the elucidation of protein structures, functions and conformational changes as well as identify novel or alternative binding sites in proteins

As an alternative to photoaffinity labeling, various techniques are known which use antibodies to bind to specific locations.

Stoeckius, M., Hafemeister, C., Stephenson, W. et al. [Nat Methods 14, 865-868 (2017)] describe cellular indexing of transcriptomes and epitopes by sequencing (CITE-seq), a method in which oligonucleotide-labeled antibodies are used to integrate cellular protein and transcriptome measurements into an efficient, single-cell readout.

Peterson, V., Zhang, K., Kumar, N. et al. [Nat Biotechnol 35, 936-939 (2017)] describe a tool to measure gene and protein expression levels in single cells with DNA-labeled antibodies and droplet microfluidics. Using the RNA expression and protein sequencing assay (REAP-seq), proteins are quantified.

Gaublomme, J.T., Li, B., McCabe, C. et al. [Nat Commun 10, 2907 (2019)] describe an approach for multiplexing snRNA-seq, using sample-barcoded antibodies to uniquely label nuclei from distinct samples.

Frei AP, Bava FA, Zunder ER, et al. [Nat Methods. 2016;13(3):269-275] describe Proximal Ligation Assay for RNA ("PLAYR") which makes use of mass spectrometry to simultaneously analyse the transcriptome and protein levels in single cells. In this technique both the proteins and RNA transcripts are labelled with isotope-conjugated antibodies and isotope-labelled probes, respectively, enabling their detection on a mass spectrometer.

There yet is a need for more versatile methods, systems, and compounds that allows selective tagging and subsequent identification of cells in a sample.

### SUMMARY

Some aspects of the present disclosure relate to methods for distinguishing cells. A sample with cells is provided with photoaffinity tags. Each tag comprises a photoreactive moiety configured to (covalently) bind to a nearby cell upon irradiation by photoactivating light, and a molecular identification structure connected to the photoreactive moiety. A target location of the sample is determined, e.g. by imaging the sample under a microscope. The target location is selected to include a target cell which is to be distinguished later. The target location is selectively irradiated with the photoactivating light to cause one or more photoactivated tags to (exclusively) bind with the target cell in the target location. The target cell can henceforward be distinguished from other (non-targeted) cells in the sample by identifying the molecular identification structure of the one or more tags (covalently) bound to the target cell. For example, by using an oligonucleotide as the molecular identification structure, many different tags can be produced which can uniquely identify different tagged cells. Advantageously, the molecular identification structure is attached to a photoreactive moiety which can be bound to any nearby cell structure - in contrast e.g. to antibodies which bind only to specific locations/proteins. Accordingly, the technique enables multiplexed photolabeling technology e.g. for linking multiple observed phenotypes of cells in a sample with respective genotypes of those cells.

Other or further aspects relate to a photoaffinity tags comprising a photoreactive moiety conjugated to a molecular identification structure. Preferably, the photoreactive moiety comprises at least one of a diazirine, a benzophenone, or an arylazide moiety. Preferably, the molecular identification structure comprises a nucleotide sequence comprising a nucleotide code sequence, most preferably a DNA or RNA nucleotide code sequence, e.g. comprising between twenty and two hundred nucleotides, and a primer sequence which preferably comprises a poly(A)-tail or comprises another primer sequence. Alternatively, or additionally, the molecular identification structure comprises an isotopic mass spectroscopy tag comprising isotope-incorporated peptide that is suitable for mass-spectrometry based proteomics.

Other or further aspects relate to methods of manufacturing photoaffinity tags for tagging a target cell in a sample. In some embodiments, the tag is manufactured by determining a genome or transcriptome sequence of nucleotides in cells of the sample, and generating an oligonucleotide with a sequence of nucleotides that is distinct from any subsequence of the genome or transcriptome sequence. Preferably, the oligonucleotide sequence is reacted with a photoreactive moiety configured to bind to a target cell upon irradiation by photoactivating light.

Other or further aspects relate to a system for tagging cells. The system comprises a sample holder configured to hold a sample with cells. Preferably, the holder is also configured to provide the sample with a respective solvent containing a respective type of photoaffinity tags. An imaging device is configured to record one or more images of the sample and a light source is configured to direct a beam of photoactivating light to selectively expose one or more target cells of the sample. Preferably, the system comprises a controller configured to provide the sample with a first solvent comprising a first type of tag, measure a first type of cellular characteristic of the cells based the one or more images, determine a first target cell based on the first type of cellular characteristic, and expose the first target cell to the photoactivating light for binding the first type of tag to the first target cell. The procedure can be repeated for other types of tags supplied to the sample. In this way many different cells can be tracked by their respective tag.

### BRIEF DESCRIPTION OF DRAWINGS

These and other features, aspects, and advantages of the apparatus, systems and methods of the present disclosure will become better understood from the following description, appended claims, and accompanying drawing wherein:
FIGs 1A-1C illustrates a method to distinguish a target cell from other cells in a sample;
FIGs 2A-2C illustrate a photoaffinity tag attaching to a nearby target cell under the influence of photoactivating light;
FIG 3A illustrates a photoaffinity tag using diazirine as photoreactive moiety;
FIG 3B illustrates a photoaffinity tag using an oligomer as molecular identification structure;
FIG 3C illustrate a method of manufacturing a photoaffinity tag;
FIG 3D illustrates a bioanalyzer used in the manufacturing;
FIG 4A illustrates a photoaffinity tag with a photoreactive moiety conjugated to a nucleotide sequence tag;
FIGs 4B - 4D illustrate diazirines forming a respective photoreactive moiety;
FIG 5 illustrates distinguishing two different tags based on their different molecular identification structure;
FIG 6A illustrates irradiating a target cell in an essentially two-dimensional sample;
FIG 6B illustrates irradiating a target cell in an essentially three-dimensional sample;
FIGs 7A-7C illustrate a method for isolating cells;
FIGs 8A-8C illustrate isolating a phototagged target cell;
FIG 9 illustrates a microscope system for imaging and phototagging samples.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

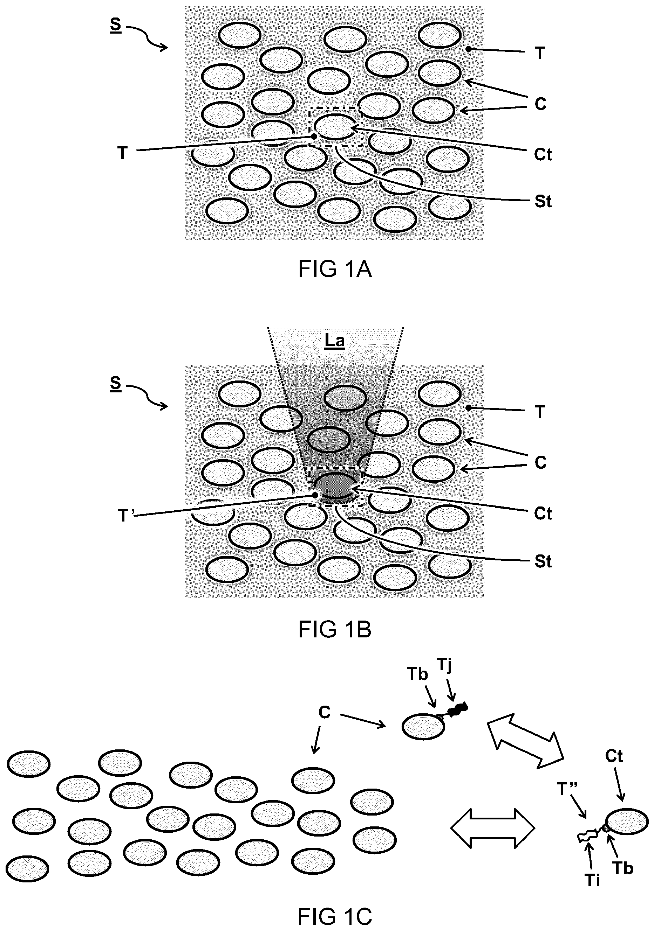

FIGs 1A-1C illustrates a method to distinguish a target cell Ct from other cells C in a sample S.

In one embodiment, a sample S with cells C, Ct is provided with photoaffinity tags T. Each tag T comprises a photoreactive moiety Tb configured to (covalently) bind to a nearby cell upon irradiation by photoactivating light La, and a molecular identification structure Ti connected to the photoreactive moiety. In some embodiments, a target location St of the sample S is determined with a target cell Ct to be distinguished. In other or further embodiments, the target location St is selectively irradiated with the photoactivating light La to cause a photoactivated tag T' to (exclusively and/or covalently) bind with the target cell Ct in the target location St. Accordingly, the target cell Ct can be distinguished from other (non-targeted) cells C in the sample S by identifying the molecular identification structure Ti of the tag T" (covalently) bound to the target cell Ct.

In some embodiments, the target location St is determined by imaging the sample S. For example, the target cell Ct in the sample S is identified based on the imaging. Accordingly, the target location St can be set to coincide with the identified target cell Ct. For example, the target cell Ct is identified based on cellular characteristics such as whether the cell is static or dynamic (e.g., aggressive migration), whether the cell is dormant (slow proliferating cells), binucleate cell division, multipolar division, micronuclei, upregulated DNA-damage repair, aberrant cell lineage, mesenchymal-like morphology, abnormal transcriptomic dynamics, irregular protein dynamics, et cetera.

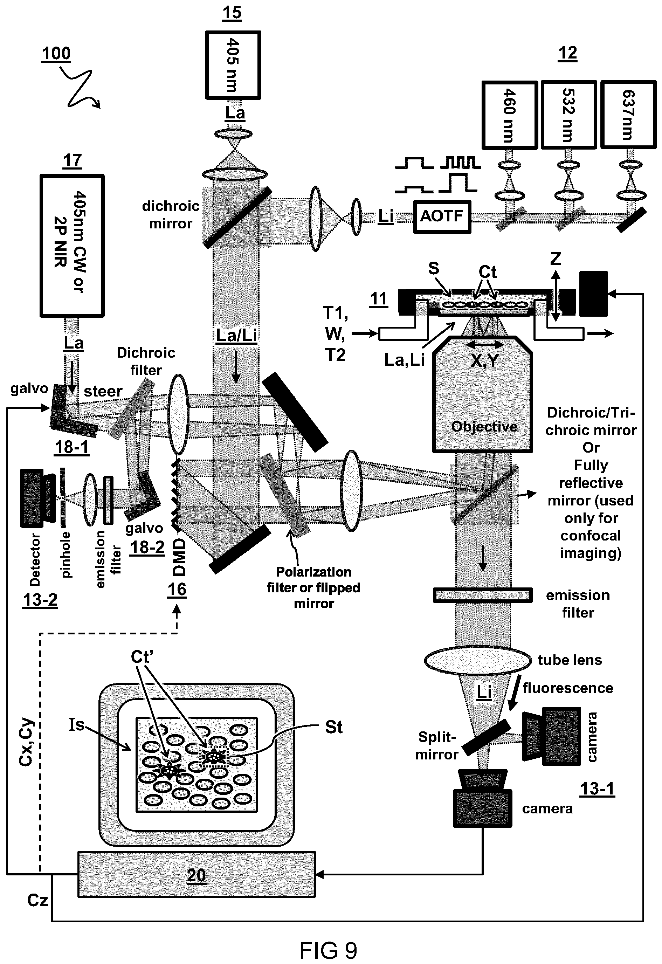

In some embodiments, the cellular characteristics are identified in an image or sequence of images representing the sample at one or more instances of time. For example, the characteristics are determined manually or automatically, e.g. based on a machine learning algorithm which is trained to recognize specific characteristics. In some embodiments, the sample S is imaged using a microscope which allows both imaging of the sample and selective radiation of a target location St. For example, coordinates of the selective irradiation are calibrated to coincide with corresponding coordinates of the imaging system. In this way a location of a target cell Ct in the image can be correlated to an actual position in the sample S for directing the beam or pattern of photoactivating light La. A preferred embodiment of a microscope system is described, e.g., with reference to FIG 9.

In some embodiments, at least some of the cells C,Ct are isolated from the sample S and respectively examined to measure or otherwise determine the molecular identification structure Ti of a respective tag T" (if it is bound to the target cell Ct). Typically most of the other cells C do not have any tag, so these can also be distinguished from the target cell Ct by the absence of a respective tag. Alternatively or additionally, some of the other cells C can have a respective tag with another molecular identification structure Tj, which can be distinguished from the molecular identification structure Ti of the tag T" attached to the target cell Ct. In principle, these other tags can have the same photoreactive moiety Tb, irrespective of the differing molecular identification structure Ti,Tj, or they can each have a different photoreactive moiety, e.g. each reacting at a respective distinct (exclusive) wavelength. For example, two or more tags can have distinct respective molecular identification structures (Ti,Tj) attached to distinct respective photoreactive moieties.

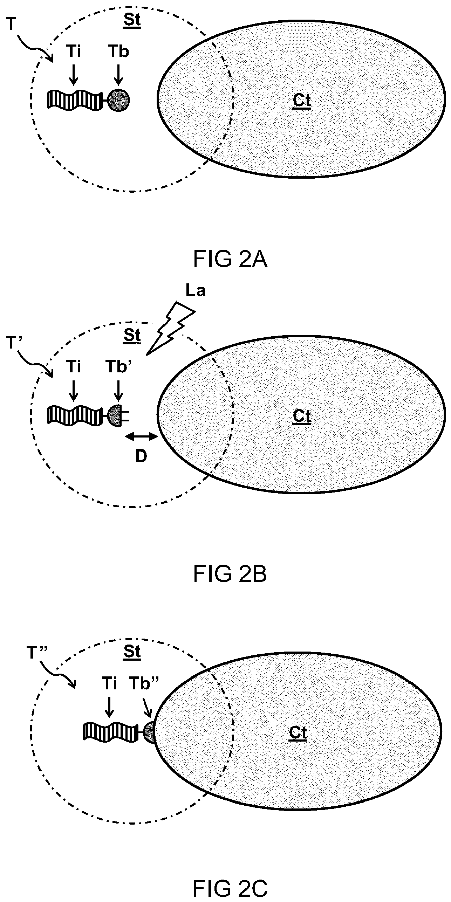

FIGs 2A-2C illustrate a photoaffinity tag T attaching to a nearby target cell Ct under the influence of photoactivating light La. It will be noted that the illustrative figures, as shown, are not to scale. For example, the tag T will typically be much smaller than the target cell Ct in the sample. It will also be noted that the present figure only shows one tag T adjacent a target cell Ct, while in general there can be many tags near the cell in the target location St.

In principle, only one tag is needed to bind to the target cell Ct, but it can also be envisaged that a plurality of tags is bound to the target cell upon irradiation of the target location St, depending on the density of tags present in the target location St. For example, irradiation may cause two, three, five, ten, twenty, fifty, hundred, or more, of the same tags to be attached to the target cell. Attaching multiple of the same tags to the target cell can help to find the molecular identification structure Ti later, e.g. having an enhance presence or signature.

Preferably, the target location St includes only the target cell Ct (not any other cells), and the photoactivating light La is exclusively applied to the target location St. Accordingly, the photoactivating light La may cause only the target cell Ct to be provided with one or more tags T of the same or different type. In some embodiments, e.g. as shown, only part of the target cell Ct is encompassed in the target location St. For example, the photoactivating light La is applied only to a subsection of the target cell Ct and/or a location immediately adjacent the target cell. While the present figure shows the target location St partly intersecting a perimeter of target cell Ct, the target location St can also be completely encompassed as a subsection of the cell perimeter. Of course it will be understood that the cell has a three dimensional structure so the tags can be all around the cell, so also in front of the cell from a point of view of the photoactivating light La. Accordingly, the light La can be directed directly at the target cell to cause tags around the target cell to attach to the target cell.

In some embodiments, the photoactivating light La is applied with a beam having a cross-section diameter (e.g. at its focus in the sample) smaller than a diameter (e.g. smallest cross-section) of the target cell Ct. In this way, the light can be used to target a specific cell, or even part of a cell. For example, a typical cell diameter is between ten and hundred micrometer, while the light beam (at the position of the target cell) can be much smaller, e.g. less than five micrometer, preferably less than two, or even less than one micrometer. In other embodiments (not shown), the beam can be larger so the target cell Ct is completely encompassed in the target location St. For example, the photoactivating light La is applied around the target cell Ct (but not on any neighboring cells).

In some embodiments, the tags are supplied in a liquid (solvent) surrounding the cells. Accordingly, the tags with the photoreactive moiety Tb can bind to an outside of the target cell Ct. Typically, the photoreactive moiety Tb as described becomes very reactive upon irradiation with the photoactivating light La. This means the tag T will typically bind to the nearest cell that is within the smallest distance from the tag when it is photoactivated. For example, the tag can be within a distance D of one micrometer or less from the cell perimeter to which it binds.

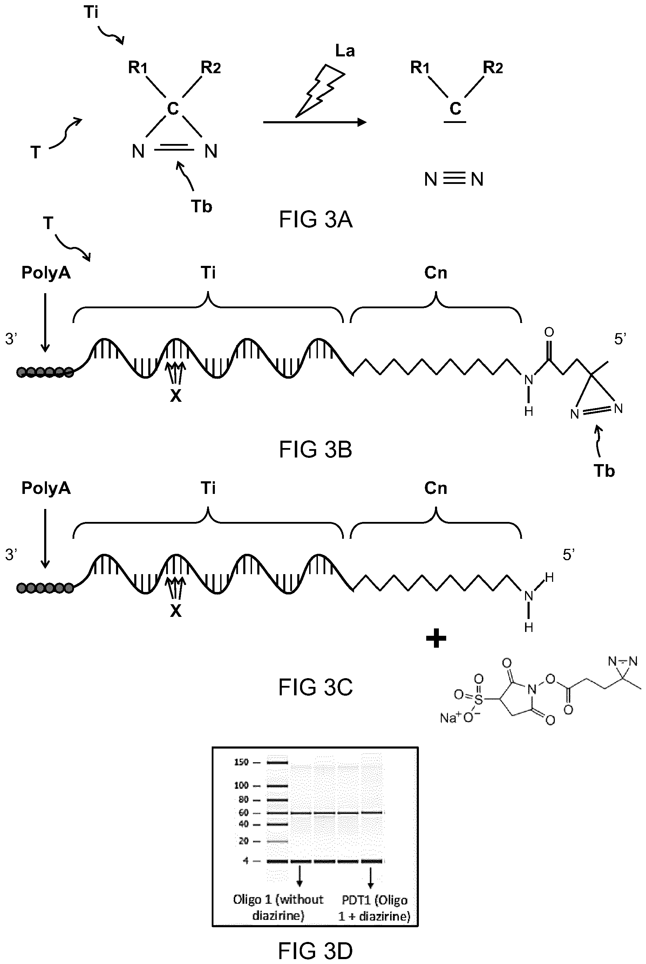

FIG 3A illustrates a photoaffinity tag T using diazirine as photoreactive moiety Tb.

Preferably, the photoreactive moiety Tb as used herein is capable of binding indiscriminately and immediately to any nearby cell structure upon selective irradiation by photoactivating light La. This may be contrasted e.g. to antibodies, which only bind to specific complementary structures. Advantageously, since the photoreactive moiety Tb can be connected to any nearby cell, no a priori knowledge of the cell is necessary in designing the tag and the tag can be attached to any desired target cell. In principle, various types of photoreactive moieties Tb can be used to bind a respective tag to a cell. In some embodiments, the photoreactive moiety Tb is configured to generate carbene radicals under the influence of the photoactivating light La, e.g. diazirine.

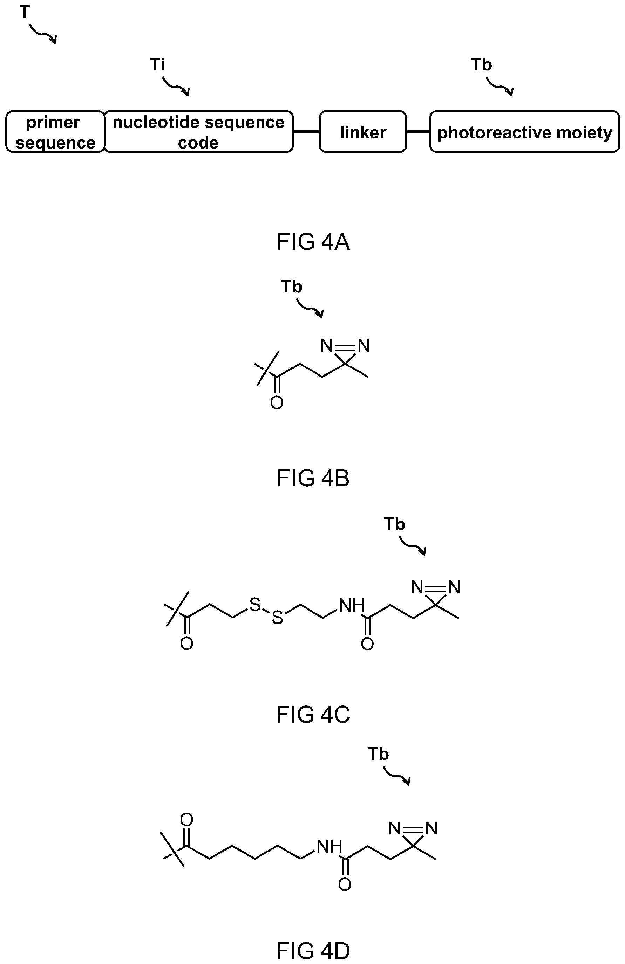

Preferably, the photoreactive moiety Tb comprises at least one of a diazirene, a benzophenone, and an arylazide moiety. For example, Dubinsky et al. [Bioorganic & Medicinal Chemistry 20 (2012) 554-570)] describes various types of photoreactive moieties. 3*H*-Diazirines (herein also referred to as diazirines) are a class of organic groups comprising a carbon bound to two nitrogen atoms, which are double-bonded to each other, forming a cyclopropene-like ring, 3H-diazirene. Upon irradiation with ultraviolet light, diazirines form reactive carbenes, which can insert into C-H, N-H, and O-H bonds. Hence, diazirine can be used as a small photoreactive crosslinking reagent. For example, suitable diazirine moieties may include aliphatic diazirines and 3-aryl-3*H*-diazirines, more specifically 3-perfluoroalkyl-3H-diazirines such as 3-trifluoroalkyl-3*H*-diazirines and 3-alkyl-3*H*-diazirines such as 3-methyl-3*H*-diazirines. *Inter alia* for preparation reasons (*vide infra*) the diazirine moieties 4,4'-azipentanoyl (FIG 4B), 6-(4,4'-azipentanamidohexanoyl (FIG 4D) and 2-[(4,4'-azipentanamido)ethyl]-1,3'-dithiopropionyl (FIG 4C) are particularly preferred as they can be connected to an amine-functionalized molecular identification structure using succinimidyl 4,4'-azipentanoate (NHS-Diazirine), succinimidyl 6-(4,4'-azipentanamido)hexanoate (NHS-LC-Diazirine) and succinimidyl 2-[(4,4'-azipentanamido)ethyl]-1,3'-dithiopropionate (NHS-SS-Diazirine) respectively. Of course it will be understood that also other chemical conjugation methods can be used to link a photoreactive moiety to a molecular identification structure, e.g. thiol-maleimide conjugation, click chemistry, Staudinger ligation, et cetera. Alternatively to diazirine, also other photoreactive moieties can be used, e.g. forming reactive carbenes or other structures capable of (indiscriminately) crosslinking to an adjacent cell. For example, other photoreactive moieties may include aryl azide or benzophenone, as described for instance in Dubinsky et al. [Bioorganic & Medicinal Chemistry 20 (2012)] and referred therein. In one embodiment, the photoreactive moiety (Tb) comprises an aryl azide such as a phenyl azide which forms nitrene radicals upon photoactivation. For example, suitable examples of phenyl azide moieties may include ortho-hydroxyphenyl azide, meta-hydroxyphenyl azide, tetrafluorophenyl azide, ortho-nitrophenyl azide, meta-nitropnhenyl azide.

FIG 3B illustrates a photoaffinity tag T using an oligomer as molecular identification structure Ti. An oligomer is understood as a molecular complex of chemicals that consists of a few repeating units (in contrast to a polymer where the number of monomers is, in principle, infinite).

In some embodiments, the molecular identification structure Ti is formed by a hetero-oligomer. A hetero-oligomer is understood as a molecular complex of chemicals that consists of a limited number of different molecular fragments (e.g. in contrast to a homo-oligomer with all the same molecular fragments). For example, the fragments are selected from at least two different macromolecules. Preferably, the molecular identification structure Ti comprises a unique set of molecular fragments, which fragments can be identified by any type of analysis, e.g. for determining molecular structure and/or weight. More preferably, the molecular identification structure Ti comprises a unique sequence of the molecular fragments, which can be identified by sequencing the structure. Most preferably, the molecular identification structure Ti comprises an oligonucleotide. An oligonucleotide - or, informally, "oligo" - is understood as a relatively short, (single) strand of nucleic acid fragments, such as DNA or RNA or nucleic acid mimics, or similar fragments of analogs of nucleic acids such as peptide nucleic acid or Morpholinos. For example, the oligonucleotide is formed by string of nucleic acid fragments "X", preferably RNA.

In some embodiments, the target cell Ct is distinguished from other cells C by determining its unique sequence of nucleotides forming the molecular identification structure Ti of the photoaffinity tag T. Preferably, the fragments are sufficiently unique to distinguish from other tags and/or from (sub)sequences of fragments which may occur in the cells to be distinguished. For example, the cell itself may contain various strands of RNA which are to be distinguished from an RNA sequence on the oligo-tag. To assure uniqueness, it is typically sufficient to include a sequence of at least twenty fragments, or (to be sure) preferably more than fifty fragments. Typically, the number of fragments in the tag can still be significantly less than naturally occurring fragments such as (messenger) RNA in the cell. For example, the number of fragments in the sequence of the tag is less than two hundred, preferably less than a hundred, e.g. between sixty and eighty fragments. In this way, the sequence can be easily manufactured while still being sufficiently unique. The fragments can, e.g., be selected according to a random or otherwise uniquely determined pattern of nucleotides or other molecular structures, which preferably should not occur in the cells.

FIG 3C illustrate a method of manufacturing a photoaffinity tag. Some aspects of the present disclosure can be embodied as methods of manufacturing a photoaffinity tag, e.g. for tagging a target cell Ct in a sample S, as described herein.

Some embodiments comprise determining a genome or transcriptome sequence of nucleotides in cells C of the sample S. Accordingly an oligonucleotide can be generated with a sequence of nucleotides that is distinct from any subsequence of the genome or transcriptome sequence. In some embodiments, the oligonucleotide sequence is generated comprising an amine-comprising linker. Also other linkers can be used, depending on the conjugation method. Preferably, the oligonucleotide sequence is conjugated with a photoreactive moiety Tb configured to (covalently) bind to a target cell upon irradiation by photoactivating light La.

In the fields of molecular biology and genetics, a genome is understood as the genetic material of an organism. It consists of DNA (or RNA in RNA viruses). The genome includes both the genes (the coding regions) and the noncoding DNA, as well as mitochondrial DNA and chloroplast DNA. The transcriptome is understood as the set of all RNA transcripts, including coding and non-coding, in an individual or a population of cells. By generating an oligo sequence that is distinct from any subsequence of the genome or transcriptome sequence, the tag can be uniquely identified.

In some embodiments, the photoreactive moiety Tb comprises a diazirene moiety, preferably an aliphatic diazirine moiety or a 3-aryl-3*H-*diazirine moiety, more preferably a 3-perfluoroalkyl-3*H*-diazirine moiety such as 3-trifluoroalkyl-3*H*-diazirine or a 3-alkyl-3*H*-diazirine such as 3-methyl-3*H*-diazirine moiety, most preferably a diazirene moiety selected from the group consisting of 4,4'-azipentanoyl(FIG 4A), 6-(4,4'-azipentanamido)hexanoyl (FIG 4D), 2-[(4,4'-azipentanamido)ethyl]-1,3'-dithiopropionyl (FIG 4C). For example, FIG 3C illustrates reacting sulfo-NHS-Diazirine with an amine-comprising linker group (Cn) connected to the oligonucleotide, which may result in the tag T as shown in FIG 3B, comprising the 4,4'-azipentanoyl photoreactive moiety. In principle, the linker can be any random chemical composition (i.e., aliphatic, including ethers such as polyglycol, or aromatic groups, with or without other organic substituents like thiol).

FIG 4A illustrates a photoaffinity tag T with a photoreactive moiety Tb conjugated to nucleotide sequence tag. Some aspects of the present disclosure can be embodied as a photoaffinity tag T comprising a photoreactive moiety Tb conjugated to a molecular identification structure Ti. In some embodiments, the molecular identification structure Ti comprises a nucleotide sequence tag. In other or further embodiments, the molecular identification structure Ti comprises an isotopic mass spectroscopy tag.

In some embodiments, the molecular identification structure comprises a nucleotide sequence comprising a nucleotide code sequence, preferably a DNA or RNA nucleotide code sequence, and a primer sequence (DNA or RNA) which preferably comprises a poly(A)-tail or another primer sequence. For example, the oligonucleotide code sequence comprises a sequence of between twenty and two hundreds of nucleotides. Typically, the nucleotides include different types of nucleotide (e.g. A,U,C,G for RNA; A,T,C,G for DNA).

A primer sequence is preferably included for the amplification of the nucleotide sequence using polymerase chain reaction (PCR) techniques. A primer sequence can be amplified together with a genome or transcriptome sequence in cells of the sample using its conjugate primer. In the embodiments of the present method that also comprise analysis of the genome or transcriptome of the target cell e.g. genome or transcriptome sequencing and accordingly comprise amplification of the genome or transcriptome of the target cell, it is preferred that the nucleotide sequence of the tag is also amplified during the amplification of the genome or transcriptome of the target cell. Amplification of the genome or transcriptome of the target cell is typically carried out using PCR techniques. In PCR of mRNA or (in vitro) transcription, oligoT primers are typically used as primers since mRNA in cells comprise a poly(A) tail. The poly(A) tail comprises multiple adenosine monophosphates; in other words, it is a stretch of RNA (or DNA) that has only adenine bases. For example, in nuclear poly(A)denylation in cells, a poly(A) tail is added to an RNA at the end of transcription to protect the RNA molecule from enzymatic degradation in the cytoplasm and aids in transcription termination, export of the RNA from the nucleus, and translation. Moreover, in PCR techniques, the poly(A) tail can be of assistance in RNA isolation, e.g. by quantitative precipitation of poly(A)-comprising RNA sequences.

In some embodiments, the tag T comprises a linker, e.g. a (linear) hydrocarbon chain Cn, e.g. a C₁₂-alkylene, between the molecular identification structure Ti and the photoreactive moiety Tb. Also other linkers can be used. For example, the chain may facilitate the preparation process. Alternatively to a nucleotide sequence tag also other types of molecular identification structure Ti can be used. For example, the molecular identification structure comprises an isotopic mass spectroscopy tag comprising isotope-incorporated peptide that is suitable for mass-spectrometry based proteomics.

FIGs 4B - 4D illustrate diazirines forming a respective photoreactive moiety Tb. In some embodiments, the photoreactive moiety comprises a diazirine, preferably an diazirine selected from the group consisting of aliphatic diazirines, 3-aryl-3*H*-diazirines, more preferably an aliphatic diazirine from the group consisting of 3-perfluoroalkyl-3*H-*diazirines such as 3-trifluoroalkyl-3*H*-diazirines, 3-alkyl-3*H*-diazirines such as 3-methyl-3*H*-diazirines, most preferably an aliphatic diazirine selected from the group consisting of 4,4'-azipentanoyl (FIG 4B), 6-(4,4'-azipentanamido)hexanoyl (FIG 4D), 2-[(4,4'-azipentanamido)ethyl]-1,3'-dithiopropionyl(FIG 4C). In other or further embodiments, the photoreactive moiety comprises an aryl azide, preferably a phenyl azide, more preferably a phenyl azide selected from the group consisting of ortho-hydroxyphenyl azide, meta-hydroxyphenyl azide, tetrafluorophenyl azide, ortho-nitrophenyl azide and meta-nitrophenyl azide.

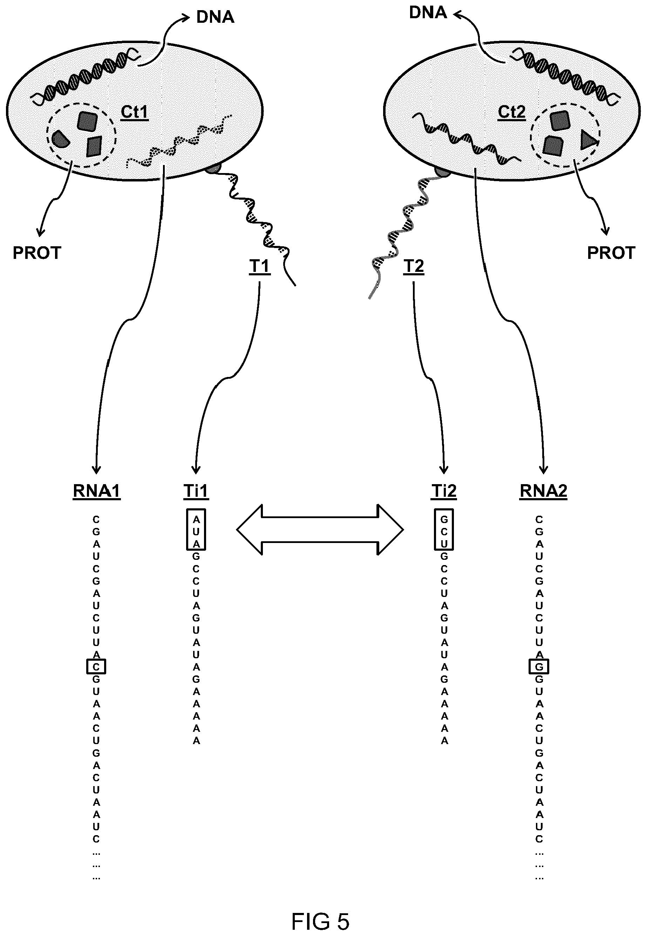

FIG 5 illustrates distinguishing two different tags T1,T2 based on their different molecular identification structure Ti.

In one embodiment, a first type tag T1 is attached by photoactivation to first target cell Ct1 in the sample, and a second type tag T2 is attached by photoactivation to different, second target cell Ct2 in the sample. The second type tag T2 can also be attached to the same target cell Ct1, e.g. when tracking different cell characteristics which are both present in the same target cell. Preferably, the first type tag T1 comprises a first molecular identification structure Ti1, and the second type tag T2 comprises a second molecular identification structure Ti2 which is structurally different from the first molecular identification structure Ti1. Accordingly, the first target cell Ct1 can be distinguished from the second target cell Ct2 based on the structural difference between the respective molecular identification structures Ti1,Ti2 of the respective tags T1,T2 attached to the respective target cells Ct1,Ct2. The first and second target cells can also be distinguished from any other (non-targeted) cell, e.g. based on the absence of a respective tag. As will be appreciated, the present methods allow the use of a virtually unlimited number of different tags that can be uniquely distinguished from each other. For example, the cells in a sample can be provided by at least two, three, four, five, ten, or more different types of tags.

Typically, most photoreactive moieties can be activated using UV light, e.g. in a wavelength range between 300 - 500 nm, e.g. 400 nm. Also other wavelengths can be used depending on the moiety. In some embodiments, different tags have different photoreactive moieties activated with photoactivating light at different wavelengths. For example, a wavelength at which a photoreactive moiety reacts can depend on the surrounding molecular structure. It can also be envisaged to used different types of photoreactive moieties, e.g. diazirene and aryl azide.

In some embodiments, the sample S is supplied with a first solvent which comprises a first type tag T1, wherein the first type tag T1 comprises a first molecular identification structure Ti1. Accordingly, the first type tag T1 can be attached to a first target cell Ct1 in the sample by photoactivation (first exposure to the photoactivating light). In other or further embodiments, the sample S is supplied with a second solvent which comprises a second type tag T2. For example, the second type tag T2 comprises a second molecular identification structure Ti2 which is different from the first molecular identification structure Ti1. Accordingly, the second type tag T2 can be attached to second target cell Ct2 in the sample by photoactivation (second exposure to the same or other photoactivating light).

Preferably, different types of tags are present in the sample at different times, which may partially overlap or not. In one embodiment, the first solvent with the first type tag T1 is removed from the sample prior to supplying the second solvent with the second type tag T2. For example, the sample is flushed in between application of different types of tags. Alternatively, it can also be envisaged to add the second type tag T2 while also keeping the first type tag T1 in the sample. For example, the second target cell Ct2 can be distinguished from the first target cell Ct1 by having both the first and second type tags T1,T2 attached to the second target cell Ct2, while the first target cell Ct1 only has the first type tag T1. On the other hand, by removing the first type tag T1 before supplying the second type tag T2, it can be uniquely determined which cells were irradiated in the presence of which respective tag. For example, a specific target cell can be irradiated twice, e.g. if it falls in two categories to be analyzed. When the first type tag T1 is removed before supplying the second type tag T2, this can be distinguished from cells which were only irradiated once while both tags T1,T2 were present.

Preferably, the molecular identification structure Ti of a respective tag T1 is uniquely identifiable, e.g. distinguishable from the molecular identification structure of another type tag T2 and from molecular structures which may be part of the cells themselves. In some embodiments, to identify a respective cell, the cell is subjected to lysis, e.g. after isolating the cell from the sample. Lysis is understood as the breaking down of the membrane of a cell, e.g. by enzymic or osmotic mechanisms that compromise its integrity. A fluid containing the contents of lysed cells is called a lysate. As described herein, the cell may be subjected to lysis in the process of purifying and/or analyzing its components such as RNA, DNA, proteins, and organelles. The molecular identification structure Ti of the tag can be adapted depending on which component of the cell is to be analyzed.

In a preferred embodiment, the respective molecular identification structure Ti comprises a sequences of nucleotides, most preferably RNA. By subjecting the cell to lysis and extracting (only) RNA components, the resulting contents can be analyzed by RNA sequencing. For example, the oligo tags can be designed with a structure that is amplified during the RNA transcription and cDNA amplification procedure of the cell contents. This can result in sequences of both the RNA of the cell, and the DNA or RNA of the tag. The latter can be used to identify the cell, e.g. link the cell to its previously observed phenotype. This phenotype can then be linked to the transcriptome using the RNA of the cell. In a similar fashion, it can also be envisaged to use a strand of DNA as the molecular identification structure, and e.g. link the phenotype of a target cell to its genome. For example, the oligo tags can be designed with a structure that is amplified during the DNA amplification procedure of the cell contents.

Alternatively to DNA and RNA, also the proteins or proteome (PROT) of a cell can be analyzed. To this end, both the proteome and the tag may be conserved while breaking down the cell. For example, the tag can be based on RNA and separately analyzed. However, this may be difficult, so also other types of tags can be envisaged. In one embodiment, the molecular identification structure comprises an isotope-labelled tag, e.g. isotope-labelled peptide tag. For example, the proteome of a cell can be analyzed using mass spectroscopy, wherein the isotope-labelled peptide tag provides a unique measure in the spectrum which can be used to identify the target cell. In the art, proteomics using mass-spectrometry is known (see e.g. Thomson et al., Analytical Chemistry 75 (2003) 1895-1904). Moreover, Tandem Mass Tag Reagents are commercially available from ThermoFisher Scientific Inc.

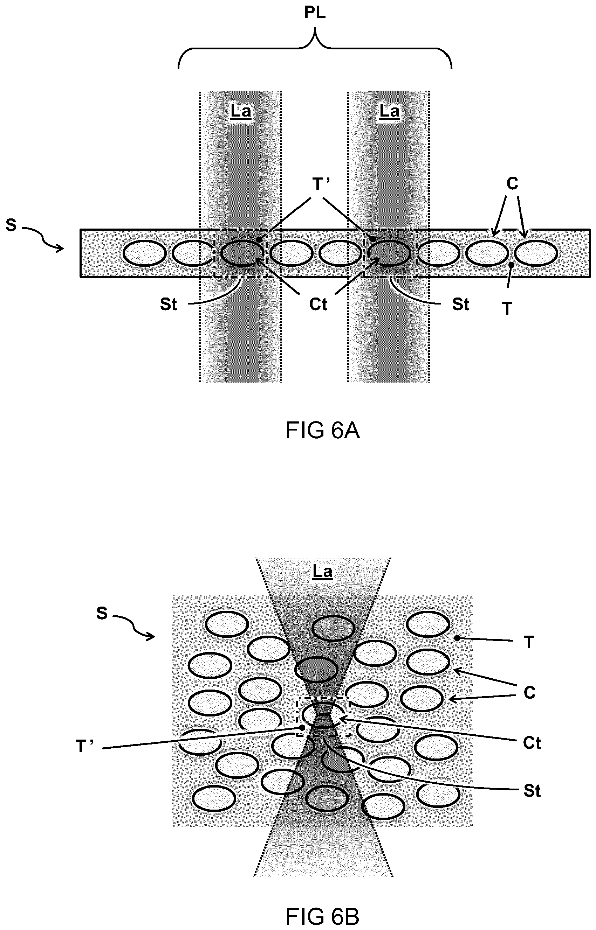

FIG 6A illustrates irradiating a target cell Ct in an essentially two-dimensional sample S.

In some embodiments, the sample S essentially consists of only a single layer of cells. This makes it relatively easy to exclusively irradiate one or more target cells Ct in the single layer, e.g. because the beam of photo-activating light La does not need to traverse other (non-targeted) cells. For example, the beam of light may be substantially directed normal to the layer of cells, as shown, or at some angle. In some embodiments, the photo-activating light La is delivered by a collimated or focused beam. For example, a width of the beam may be relatively small or on the order of the target cell size.

In some embodiments, a light pattern PL is projected onto multiple target cells. For example, the light pattern comprises multiple (disjointed) light spots at different positions of the sample. In this way multiple target cells may be simultaneously activated for subsequent isolation. In some embodiments, the light pattern is projected by multiple light beams, e.g. using beam splitters. In another or further embodiment, the light pattern may be projected by masking one or more light beams. In a preferred embodiment, as described in further detail below, the light pattern is produced by a digital mirror device (DMD) or other opto-electromechnical devices. Also other devices, such as galvanometer mirrors or Galvo mirrors, can be used to direct the light to one or more spots. In some embodiments, the sample is an essentially two dimensional sample with a single layer of cells and the photoactivatable fluorophore is activated by applying a pattern of the photo-activating light onto the sample.

FIG 6B illustrates irradiating a target cell Ct in an essentially three-dimensional sample S;

In some embodiments, a beam of the photo-activating light La is focused onto one or more target cell Ct in a two or three dimensional sample S. The light beam may be relatively small at the focal spot so the photo-activating light La can be better targeted onto the target cell Ct without irradiating surrounding cells. Also, a light intensity of the photo-activating light La at a focal point of the beam may be relatively high. So the photo-activation may be more prominent at the focal point. To further increase intensity and/or to obtain selective optical sectioning activate specific cells in three-dimensional samples, the photo-activating light La may be generated by a pulsed light source, such as a pulsed or femtosecond laser. For example, the momentary light intensity during a laser pulse can be much higher than in a continuous wave laser.

In some embodiments, an increased light intensity produced by a focal spot, laser pulse, or combination thereof, may enable unique processes to occur which do not take place at lower intensities. For example, the relatively high intensity may enable multi-photon absorption. It will be appreciated that this can be used to select the target location St of one or more cell in three dimensions. For example, two coordinates may be determined by adjusting a position or direction of the beam, and one coordinate may be determined by adjusting the beam focal position (along the beam direction). Instead of a focused light spot a locally increased intensity may also be achieved, e.g. crossing two or more beams at a target position in the sample S. In some embodiments, the sample is an essentially three dimensional sample with multiple layers of cells along a beam direction. In other or further embodiments, the photoaffinity tag T (and/or photoactivatable fluorophore as described later) is activated by two-photon or multi-photon absorption exclusively at a focal spot of the photo-activating light La, e.g. in a subset of one or more (not all) of the layers.

In one embodiment, the photo-activating light La may comprise a source wavelength λs (e.g. output from a light source) that is above a maximum activation wavelength λa at which the tag and/or agent can be activated. In a further embodiment, the source wavelength λs is below an integer multiple, e.g. double, the maximum activation wavelength, i.e. λs ≤ 2·λa, so the tag and/or agent can still be activated, e.g. by two photon absorption. For example, photo-activating light La is produced by a focal spot of an eight hundred nanometer pulsed laser, where the tag and/or agent is activated by four hundred nanometer continuous wave CW light half the wavelength, double the energy. In some embodiments, the tag and/or fluorophore comprises a photosensitizer. Accordingly this may allow the use of photo-activating light La in a (visible) wavelength range, e.g. above 400 nm.

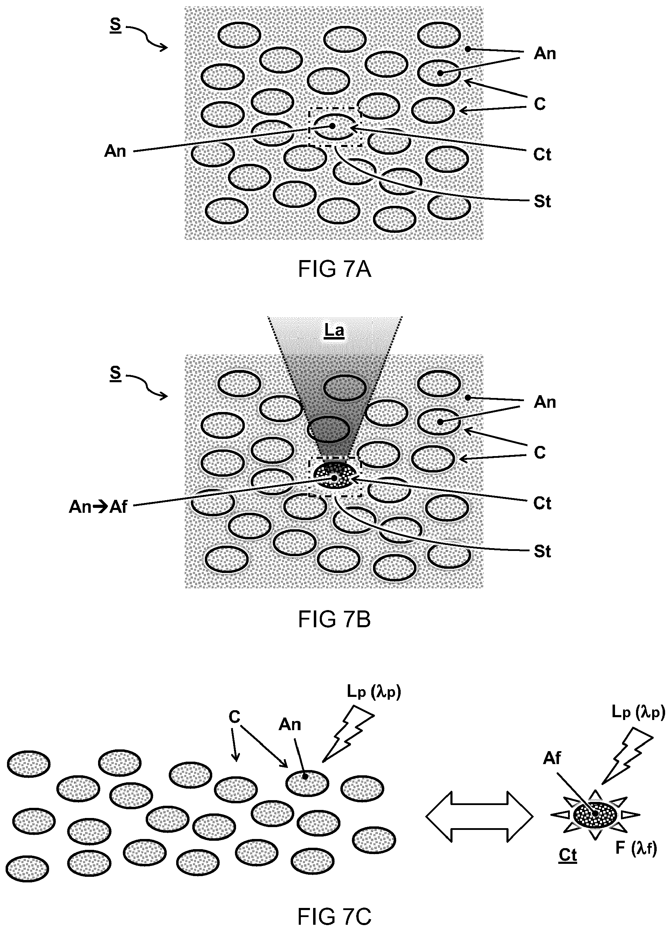

FIGs 7A-7C illustrate a method for isolating cells. In some embodiments, the cells in the sample S comprise a photoactivatable fluorophore An configured to change its fluorescence response upon irradiation by the photoactivating light La. Advantageously, the photo-activating light La can be used for both activating the photoactivatable fluorophore An inside the target cell Ct and the photoaffinity tag T on an outside of the target cell Ct, e.g. in a solvent surrounding the target cell. Accordingly, when the photoactivating light La is directed to irradiate a target cell Ct this can cause the target cell Ct to be labeled both by the changed fluorescence response as well as the unique tag. In some embodiments, the target cell Ct is isolated from other cells C in the sample S based on a difference in its fluorescence response F, e.g. compared to non-activated photoactivatable fluorophore "An" in the other cells C. A preferred embodiment comprises illuminating cells C from the sample S with probe light Lp, measuring a fluorescence response F of the illuminated cells C, and isolating a set of target cells Ct from other cells C in the sample S based on a difference in the fluorescence response F of the target cell Ct compared to the other cells C. Advantageously, the isolated target cells Ct can be distinguished based on different tags T1,T2 attached to the respective target cells Ct, as described earlier.

In a preferred embodiment, the activation causes a structural change An→Af of the photoactivatable fluorophore "An" contained in the target cell Ct. In some embodiments, as described herein, the activating of the photoactivatable fluorophore "An" causes an increase in its fluorescence response F. For example, the activation converts the photoactivatable fluorophore from an initially non-fluorescent molecule "An" to a fluorescent molecule "Af', or from a relatively low-fluorescent to a relatively high-fluorescent molecule. Accordingly, the target cell Ct may be isolated based on its relatively high fluorescence response F compared to the other cells. It can also be envisaged that the activation causes a decrease in the fluorescence response F of the photoactivatable fluorophore "An". In this case, the target cell Ct may be isolated based on its relatively low fluorescence response F compared to the other cells. It can also be envisaged that the activation causes higher or lower fluorescence response in photoactivated or photoconverted fluorescent proteins acting as the photoactivatable fluorophore.

In a preferred embodiment, the change in the fluorescence response F caused by the activation or conversion is substantially permanent, or at least sufficiently long lasting to allow the subsequent isolating of the target cell Ct based on the difference in the fluorescence response F. For example, the photoactivatable fluorophore "An" comprises a caged fluorophore, wherein the photo-activating light La causes an uncaging of the fluorophore, as described herein. The uncaging may permanently increase the subsequent fluorescence response F of the activated fluorophore. Also other ways may be envisaged to activate a fluorophore "An" to cause permanent or long lasting change of its fluorescence response F. Preferably, the change in response lasts more than one second, more than five seconds, more than ten seconds, more than one minute, more than five minutes, or longer, e.g. indefinitely. The longer the change in fluorescence response can last, the more time this gives between the activation/selection and subsequent isolation based on the changed response. While the preferred use of a caged fluorophore most preferably a photosensitized variant can have particular advantages, it will be appreciated that the single cell selection pipeline as described herein can also work with at least some of the same or similar advantages for any other or further kind of photoactivatable fluorophore or protein, photo-converted protein, et cetera.

In one embodiment, the photo-activating light La predominantly or exclusively activates the photoactivatable fluorophore (and photoaffinity tag) in the one or more identified target cells Ct with minimal or no activation of the photoactivatable fluorophore in other surrounding cells. For example, more than fifty percent of the photoactivatable fluorophore in the sample S being activated by the photo-activating light La is contained within the identified one or more target cells Ct, preferably at least eighty or ninety percent, or even up to a hundred percent. The more the activation is limited to the photoactivatable fluorophore contained in the target cell, the easier may be its subsequent isolation based on the distinct fluorescence response F. Of course, even with less than perfect activation, it may still be possible to isolate the target cell Ct based on its relative fluorescence response e.g. higher intensity compared to other, lesser activated, cells.

In a preferred embodiment, the photoactivatable fluorophore "An" is cell-permeable, at least before it is activated. This allows the photoactivatable fluorophore "An" to be easily added to any sample with cells, e.g. without damaging the cells. In another or further embodiment, a cell-permeable or impermeable photoactivatable fluorophore "An" is produced in the cells C. For example, the photoactivatable fluorophore "An" may be an already occurring substance in the cells, or the cells may be modified to produce the photoactivatable fluorophore "An" and/or this may be a photoactivated or photoconverted fluorescent protein. It can also be envisaged that a permeable or impermeable photoactivatable fluorophore "An" is forced, e.g. injected, into the cells. In some embodiments, the photoactivatable fluorophore loses some or all of its cell-permeability after it is activated. While not necessary, this may help to retain the activated fluorophore in the cell during isolation.

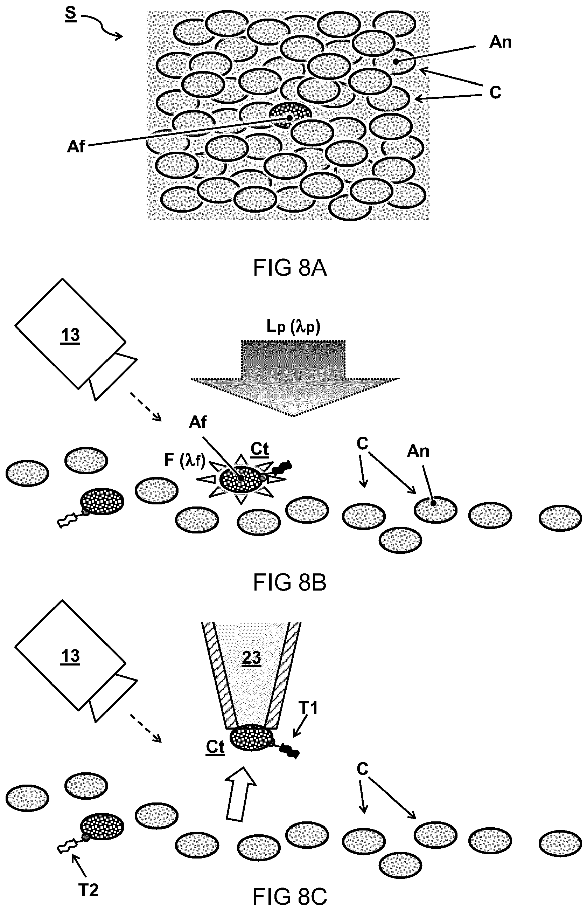

FIGs 8A-8C illustrate isolating a phototagged target cell Ct. In some embodiments, isolating the target cell Ct comprises illuminating cells C from the sample S with probe light Lp. In other or further embodiments, the fluorescence response F of the illuminated cells C is measured. In other or further embodiments, the target cell Ct is isolated based on a difference in the fluorescence response F of the target cell Ct compared to other cells C in the sample S.

Fluorescence is generally understood as a form of luminescence. Typically the fluorescence response F may manifest as the emission of light by a substance here: the activated photoactivatable fluorophore Af that has absorbed light or other electromagnetic radiation (probe light Lp). Typically, the emitted light corresponding to the fluorescence response F has a longer wavelength λf, and therefore lower energy, than a wavelength λp of the absorbed radiation, e.g. probe light Lp.

In some embodiments, a relative intensity of the fluorescence response F is used to distinguish the activated photoactivatable fluorophore Af from the non-activated photoactivatable fluorophore An. In other or further embodiments, the fluorescence wavelength λf of the fluorescence response F is used to distinguish the activated photoactivatable fluorophore Af from the non-activated photoactivatable fluorophore An. For example, the fluorescence wavelength λf may be in a distinct wavelength range from other light, e.g. other fluorescent molecules in the sample. Of course also combinations are possible wherein both wavelength and intensity are used to distinguish and isolate the cells after phototagging.

In some embodiments, the cells comprise a fluorescent compound in addition to the photoactivatable fluorophore An. This may allow relatively easy imaging of the cells, e.g. using a fluorescence microscope, before and after the phototagging. This further compound preferably has a distinct fluorescence wavelength compared to the activated photoactivatable fluorophore Af. For example, the cells may comprise both a molecule such as GFP (which emits a green fluorescent response); and a caged rhodamine-based photoactivatable fluorophore which emits a red fluorescent response, but only when uncaged or photoactivated by phototagging.

In some embodiments, the fluorescence response F is measured by a sensor or dual sensors 13, e.g. as shown in FIG 9, (imaging) camera 13-1 or any other imaging or non-imaging light sensor 13-2. Alternatively, or additionally, the fluorescence response F can be observed by eye, e.g. via a microscope (not shown). In some embodiments, the target cell Ct is isolated by physically separating the target cell Ct from the rest of the sample S. For example, a pickup tool 23 can be used to pick up and isolate the target cell Ct. In a preferred embodiment, the isolating of target cells is automated, e.g. using a pickup tool 23 configured to automatically pick up one or more target cells Ct from a sample S based on their distinctive fluorescence response F measured by a sensor 13. Also other mechanisms for isolating cells can be envisaged. For example, the cells may be guided through a flow channel and sent in different directions depending on their fluorescence response F.

In some embodiments, the sample S is disintegrated, e.g. the sample is dissolved/dissociated and/or the cells are separated from each other. This can make it easier to isolate the target cell Ct, e.g. in a three dimensional sample. In some embodiments, the cells C of the sample S are spread out on a substrate. For example, the pickup tool 23 can be used to pick the cell from the substrate. Alternatively, the spreading is already sufficient and the isolating comprises identifying the target cell Ct among the other cells.

After one or more target cells are identified and/or isolated based on the respective fluorescence response, compared to other cells, the one or more target cells can be further analyzed to determine the respective tags T1,T2. While the present figures illustrate isolating one or more target cells based on fluorescence response, it can also be envisaged to isolate, or otherwise identify the target cells based only on the respective tags T1,T2 attached to the respective cells, as described earlier.

FIG 9 illustrates a microscope system 100 for imaging and phototagging samples.

In a preferred embodiment, the microscope system 100 is configured to perform the methods as described herein. In some embodiments, a sample holder 11 is configured to hold a sample S with cells C containing a photoactivatable fluorophore An and/or the photoaffinity tags T1,T2 as described herein. One embodiment of a system for tagging cells comprise a sample holder configured to hold a sample with cells and provide the sample S with a respective solvent containing a respective type of photoaffinity tags T1,T2. Preferably, the sample holder 11 comprises a flow cell configured to flush the sample with respective solvents. For example, the sample can be respectively flushed with different solvents comprising respective tags T1,T2. Optionally, a clean solvent such as buffer or water W can be used to remove the previous tags after illumination.

In some embodiments, the system comprises an imaging device configured to record one or more images of the sample S, e.g. a microscope. In another or further embodiment, the system comprises a light source configured to direct a beam of photoactivating light La to selectively expose one or more target cells Ct of the sample S. In some embodiments, the system comprises a controller to perform operational acts in accordance with the present methods. In one embodiment, the controller is configured to provide the sample with a first solvent comprising a first type of tag, measure a first type of cellular characteristic of the cells based the one or more images, determine a first target cell based on the first type of cellular characteristic, and expose the first target cell to the photoactivating light for binding the first type of tag to the first target cell. For example, the cellular characteristics can be distinguished manually or automatically, e.g. using a machine learning algorithm. In another or further embodiment, the controller is configured to provide the sample with a second solvent comprising a second type of tag, measure a second type of cellular characteristic of the cells based on the one or more images, determine a second target cell based on the second type of cellular characteristic, and expose the second target cell to the photoactivating light for binding the second type of tag to the second target cell.

In some embodiments, one or more image light sources 12 (or 17) are configured to illuminate the sample with imaging light Li. In other or further embodiments, at least one light detector (e.g. 13-1,13-2) is configured to detect a sample image Is of the illuminated sample S for spatially resolving the cells C. In other or further embodiments, one or more photo-activation light sources 15, 17 configured to generate photo-activating light La for activating the photoactivatable fluorophore to change its fluorescence response F.

In a preferred embodiment, a controller 20 is configured to determine a target selection St of an imaged target cell Ct' based on the sample image Is, and determine target coordinates Cx,Cy of a corresponding target cell Ct in the sample S based on the target selection St. In other or further preferred embodiments, beam patterning and/or steering optics 16,18 are configured to receive the target coordinates Cx,Cy of the target cell Ct, and selectively direct the photo-activating light La to irradiate a target location X,Y of the target cell Ct in the sample S based on the target coordinates Cx,Cy.

In one embodiment, target coordinates Cx,Cy,Cz of the target cell Ct in the sample S are determined based its identification in the imaging. For example, the image comprises a two or three dimensional mapping of cells in the sample. Positions of the cells in the image may thus be correlated mapped to their actual positions in the sample. Accordingly, the position of a cell selected on the basis of the imaging may be used to determine its location in the sample and the photo-activating light La can be selectively directed to the target location St. For example, a combined optical system can be used for both imaging and phototagging the cells, as described herein. The system may thus be calibrated to direct a position of the photo-activating light La to an imaged target position St in the sample S.

In some embodiments, at least one of the photo-activation light sources comprises a pulsed laser 17 which is focused at a specific location inside the sample S for photo-activating the respective photoaffinity tag T and/or photoactivatable fluorophore An by two-photon absorption. For example, lenses or mirrors can be used to focus the beam. As explained before with reference to FIG 5B, focused laser pulses may allow to activate photoactivatable fluorophore at a specific depth in the sample using two-photon absorption which is limited to the focal region where the light intensity can be relatively high. In a preferred embodiment, the sample holder 11 comprises a moveable stage configured to vary a coordinate Z of the laser focus in the sample along an optical axis of the microscope based on at least one of the target coordinates Cz of the target cell Ct. Alternatively, or additionally, a depth of the focal position may be varied, e.g. by adjusting the lenses.

In some embodiments, the system comprises a light patterning device 16 in a light path between one or more continuous wave lasers or LED light emitting diode 12,15 and the sample holder for patterning the photo-activating light La and/or imaging light Li onto the sample S. In other or further embodiments, the light patterning device 16 is arranged in a light path between at least one of the photo-activation light sources 15 and the sample holder 11 for selectively applying the photo-activating light La onto the selected target cell Ct of an essentially two-dimensional sample S.

In a preferred embodiment, the system is configured for wide-field imaging of the sample. Most preferably, the microscope offers the ability to image with both high resolution and large FOV, both with one photon and two photon excitation, and in both two-dimensional 2D and three-dimensional 3D samples. By imaging a large part of the sample at once, multiple cells can be simultaneously imaged and tracked. In some embodiments, the light patterning device 16 is arranged in a light path between at least one of the image light sources 12 and the sample holder 11 for applying a pattern of the imaging light Li.

In a preferred embodiment, the system is configured for (fast) volumetric imaging reconstruction of 3D samples using wide-field or scanning imaging methods allowing planar section, including but not limited to scanning two-photon microscopy, structured illumination microscopy or Hadamard imaging. In general, the system can be configured for 2D or 3D image reconstruction using any illumination method and image reconstruction method that can be used to create imaging contrast, including but not limited to white-light imaging, phase-contrast imaging, bright-field imaging, dark-field imaging, (wide-field) fluorescence imaging or scanning confocal imaging.

In some embodiments, a three dimensional image or map of the sample S is recorded or registered, e.g. using 3D-structured illumination microscopy, multi-photon imaging or any other suitable technique such as confocal microscopy. Also other or further devices may be arranged in a light path between one or more light of the image light sources 12 and the sample holder 11. For example, an acousto-optic tunable filter (AOTF) can be used to rapidly and dynamically select a specific wavelength from a broadband or multi-line laser source, as shown. For example, different fluorescence images of the sample S may be taken by illuminating at different wavelengths. Multi-line light sources can include any desired wavelengths of light, not limited to the shown 460, 532 or 637nm.

In some embodiments, imaging of the sample can be done either in widefield or confocal configuration by switching the dichroic/trichroic filter in the emission path to a fully reflective mirror. As will be appreciated, advantages of the present disclosed microscope systems may include imaging a large quantity of cells (e.g. hundreds thousands of or ∼millions of cells) with high spatial (sub-cellular; < micrometer) and temporal (microseconds, milliseconds, seconds or minutes depending on users' preference) resolution. To facilitate capturing such a large quantity of cells without losing temporal resolution, dual detectors (e.g. cameras 13-1) can be implemented in some embodiments, which can simultaneously capture dual fields of view. To facilitate imaging finite sub-cellular structures that are below the spatial resolution of the microscope's standard operation, an additional moiety can be implemented in the setup and can be chosen if needed, e.g. switching the dichroic/trichroic mirror at the emission path to a fully reflective mirror so that confocal imaging can be performed. Confocal imaging implemented in the setup can yield two to several folds of improvement in terms of spatial resolution (e.g. from 0.8 micrometer to 0.4 micrometer with a twofold improvement).

In some embodiments, the imaging setup may include one or more sets of galvo mirrors (e.g. 18-2). For example, this can be used for confocal imaging instead of, or in addition to, wide-field imaging. Also other or further components can be included in the imaging path, e.g. a pinhole and/or emission filter. While the present system shows a second galvo mirror 18-2, it can also be envisaged to (re)use the same galvo mirror 18-1 for the same or similar purpose in other embodiments (not shown).

For the purpose of clarity and a concise description, features are described herein as part of the same or separate embodiments, however, it will be appreciated that the scope of the invention may include embodiments having combinations of all or some of the features described. In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage.

The invention can further be illustrated with the following nonlimiting example.

### EXAMPLE 1 - Preparation of photoaffinity tags

Photoaffinity tags were produced as follows. To a 1.5 mL eppendorf tube containing 25 nmoles of oligos (based on poly A, molecular identification oligos, C 12 linker and an amine group) in PBS buffer was added 100 nmoles of sulfo-NHS-Diazirine. The reaction was incubated at room temperature for 6 min. Addition of sulfo-NHS-Diazirine was repeated another 4 times with incubation of 6 min/addition to ensure a successful conjugation of sulfo-NHS-Diazirineto the oligos. The reaction was then quenched by adding Tri-HCl (pH 8.0) to a final concentration of 50-100 mM for 5 min at room temperature. The product was then desalted with a Bio-Spin P6 column according to manufacturer's protocol. The conjugated product was then validated using an Agilent BioAnalyzer Small RNA chip, as shown in FIG 3D.

## Claims

1. A method for distinguishing cells, the method comprising
providing a sample (S) with cells (C,Ct) and photoaffinity tags (T), wherein each tag (T) comprises
a photoreactive moiety (Tb) configured to bind to a nearby cell upon irradiation by photoactivating light (La), and
a molecular identification structure (Ti) connected to the photoreactive moiety;
determining a target location (St) of the sample (S) with a target cell (Ct) to be distinguished;
selectively irradiating the target location (St) with the photoactivating light (La) to cause a photoactivated tag (T') to bind with the target cell (Ct) in the target location (St); and
distinguishing the target cell (Ct) from other cells (C) in the sample (S) by identifying the molecular identification structure (Ti) of the tag (T") bound to the target cell (Ct).

2. The method according to the preceding claim,
wherein a first type tag (T1) is attached, by a first exposure of the photoactivating light (La), to a first target cell (Ctl) in the sample, and a second type tag (T2) is attached, by a second exposure of the photoactivating light (La), to different, second target cell (Ct2) in the sample;
wherein the first type tag (T1) comprises a first molecular identification structure (Ti1), and the second type tag (T2) comprises a second molecular identification structure (Ti2) that is structurally different from the first molecular identification structure (Ti1);
wherein the first target cell (Ctl) is distinguished from the second target cell (Ct2) based on the structural difference between the respective molecular identification structures (Ti1,Ti2) of the respective tags (T1,T2) attached to the respective target cells (Ct1,Ct2)

3. The method according to the preceding claim, comprising
supplying the sample (S) with a first solvent which comprises the first type tag (T1);
attaching the first type tag (T1) to first target cell (Ctl) in the sample by the first exposure;
supplying the sample (S) with a second solvent which comprises the second type tag (T2); and
attaching the second type tag (T2) to second target cell (Ct2) in the sample by the second exposure.

4. The method according to the preceding claim, wherein the first type tag (T1) is removed from the sample prior to supplying the second type tag (T2).

5. The method according to any of the preceding claims, wherein the cells in the sample (S) comprise a photoactivatable fluorophore (An) configured to change its fluorescence response upon irradiation by the photoactivating light (La).

6. The method according to the preceding claim, comprising
illuminating cells (C) from the sample (S) with probe light (Lp); measuring a fluorescence response (F) of the illuminated cells (C);
isolating a set of target cells (Ct) from other cells (C) in the sample (S) based on a difference in the fluorescence response (F) of the target cell (Ct) compared to the other cells (C); and
distinguishing between the isolated target cells (Ct) based on different types of tags (T1,T2) attached to the respective target cells (Ct).

7. The method according to any of the preceding claims, wherein the target location (St) is determined by
imaging the sample (S);
identifying the target cell (Ct) in the sample (S) based on cellular characteristics observed in the imaging; and
setting the target location (St) to coincide with the identified target cell (Ct).

8. The method according to any of the preceding claims, wherein the photoactivating light (La) is applied with a beam having a cross-section diameter smaller than a diameter of the target cell (Ct).

9. The method according to any of the preceding claims, wherein the photoreactive moiety (Tb) comprises at least one of a diazirene, a benzophenone, and an aryl azide moiety, preferably a diazirine moiety.

10. The method according to any of the preceding claims, wherein the molecular identification structure (Ti) comprises an oligonucleotide; and the target cell (Ct) is distinguished from other cells (C) by determining its unique sequence of nucleotides forming the molecular identification structure (Ti) of the photoaffinity tag (T).

11. The method according to any of the preceding claims, wherein the molecular identification structure (Ti) comprises an isotopic mass spectroscopy tag comprising isotope-incorporated peptide; and the target cell (Ct) is distinguished from other cells (C) by mass-spectrometry based proteomics.

12. A photoaffinity tag (T) comprising a photoreactive moiety (Tb) conjugated to a molecular identification structure (Ti),
wherein the photoreactive moiety comprises at least one of a diazirine, a benzophenone, or an arylazide moiety;
wherein the molecular identification structure (Ti) comprises
a nucleotide sequence comprising a nucleotide code sequence, preferably a DNA or RNA nucleotide code sequence of between twenty and two hundred nucleotides, and a primer sequence which preferably comprises a poly(A)-tail or another primer sequence, or
an isotopic mass spectroscopy tag comprising isotope-incorporated peptide that is suitable for mass-spectrometry based proteomics.

13. A method of manufacturing a photoaffinity tag for tagging a target cell (Ct) in a sample (S), the method comprising
determining a genome or transcriptome sequence of nucleotides in cells (C) of the sample (S);
generating an oligonucleotide with a sequence of nucleotides that is distinct from any subsequence of the genome or transcriptome sequence; and
reacting the oligonucleotide sequence with a photoreactive moiety (Tb) configured to bind to a target cell upon irradiation by photoactivating light (La).

14. A system for tagging cells, the system comprising
a sample holder configured to hold a sample with cells and provide the sample (S) with a respective solvent containing a respective type of photoaffinity tags (T1,T2);
an imaging device configured to record one or more images of the sample (S);
a light source configured to direct a beam of photoactivating light (La) to selectively expose one or more target cells (Ct) of the sample (S); and
a controller configured to
provide the sample (S) with a first solvent comprising a first type of tag (T1),
measure a first type of cellular characteristic of the cells based on the one or more images,
determine a first target cell (Ctl) based on the first type of cellular characteristic, and
expose the first target cell (Ctl) to the photoactivating light (La) for binding the first type of tag (T1) to the first target cell (Ct1).

15. The system according to the preceding claim, wherein the sample holder is a flow cell configured to selectively flush the sample with different solvents comprising respective photoaffinity tags (T1,T2), wherein the controller is further configured to
provide the sample (S) with a second solvent comprising a second type of tag (T2),
measure a second type of cellular characteristic of the cells based the one or more images, which is a different characteristic than the first type of cellular characteristic,
determine a second target cell (Ct2) based on the second type of cellular characteristic, and
expose the second target cell (Ctl) to the photoactivating light (La) for binding the second type of tag (T2) to the second target cell (Ct2).
